(19) Europäisches Patentamt
European Patent Office
Office européen des brevets

(11) **EP 3 839 963 B1**

(12) **EUROPEAN PATENT SPECIFICATION**

(45) Date of publication and mention
of the grant of the patent:
**15.04.2026 Bulletin 2026/16**

(21) Application number: **19218555.1**

(22) Date of filing: **20.12.2019**

(51) International Patent Classification (IPC):
**G16H 20/10** $^{(2018.01)}$        **G16H 20/17** $^{(2018.01)}$

(52) Cooperative Patent Classification (CPC):
**G16H 20/10; G16H 20/17**

(54) **DIABETES ANALYSIS SYSTEM, AND METHOD IN RELATION TO THE SYSTEM**

DIABETESANALYSESYSTEM UND VERFAHREN IM ZUSAMMENHANG MIT DEM SYSTEM

SYSTÈME D'ANALYSE DU DIABÈTE ET PROCÉDÉ EN RELATION AVEC LE SYSTÈME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**23.06.2021 Bulletin 2021/25**

(73) Proprietor: **Digital Diabetes Analytics Sweden AB
112 56 Stockholm (SE)**

(72) Inventors:
• **CEDERBLAD, Lars
  723 49 Västerås (SE)**
• **ESPES, Daniel
  752 17 Uppsala (SE)**
• **CARLSSON, Per-Ola
  743 40 S Storvreta (SE)**

(74) Representative: **Bjerkén Hynell KB
P.O. Box 1061
101 39 Stockholm (SE)**

(56) References cited:
WO-A1-2015/025187    WO-A1-2018/175935
KR-A- 20190 004 733    US-A1- 2011 040 204

US-A1- 2012 165 638    US-A1- 2013 035 871
US-A1- 2015 347 698    US-A1- 2016 354 543

• ANONYMOUS: "Outlier", WIKIPEDIA, 18 December 2019 (2019-12-18), XP055698794, Retrieved from the Internet <URL:https://en.wikipedia.org/w/index.php?title=Outlier& oldid=931339528> [retrieved on 20200527]
• D. M. NATHAN ET AL: "Translating the A1C Assay Into Estimated Average Glucose Values", DIABETES CARE, vol. 31, no. 8, 7 June 2008 (2008-06-07), pages 1473 - 1478, XP055099114, ISSN: 0149-5992, DOI: 10.2337/dc08-0545
• BAYSAL NIHAT ET AL: "A Novel Method to Detect Pressure-Induced Sensor Attenuations (PISA) in an Artificial Pancreas", JOURNAL OF DIABETES SCIENCE AND TECHNOLOGY, vol. 8, no. 6, 14 October 2014 (2014-10-14), US, pages 1091 - 1096, XP093346744, ISSN: 1932-2968, Retrieved from the Internet <URL:https://journals.sagepub.com/doi/epub/10.1177/1932296814553267> DOI: 10.1177/1932296814553267

EP 3 839 963 B1

## Description

Technical field

The present disclosure relates to a diabetes analysis system, and method in relation to the system. In particular, the disclosure relates to automated analysis and interpretation of glucose related data.

Background

Type 1 diabetes (T1D) is the most common chronic disease in children, for which there is no cure and at least 800 children are diagnosed every year in Sweden alone. Although at a lower incidence, T1D can also debut later in life in all age groups. Individuals diagnosed with T1D will for the rest of their lives entirely depend on exogenous insulin. In addition to daily insulin injections, multiple blood glucose testing is required in order to correctly dose insulin. However, exogenous insulin treatment is by no means able to fully mimic the fine-tuned regulation of insulin secretion in healthy individuals, and individuals with T1D are therefore at constant risk of death or brain damage due to dangerously low glucose levels (GL) (hypoglycemia). Studies have shown that 6-10% of all deaths among patients with T1D is caused by hypoglycemia. At the same time, the inevitable mean increase in blood glucose concentrations dramatically increases the risk of long-term complications, which results in a reduction of life-expectancy in T1D by more than 10 years.

Over the last few years, the technology for continuous glucose monitoring (CGM) and flash glucose monitoring (FGM) has dramatically improved, and many countries now reimburse patients for the use of CGM/FGM. In flash glucose monitoring, patients have a sensor inserted on their upper arm and a separate touchscreen reader device. When the reader device is swiped close to the sensor, the sensor transmits both an instantaneous glucose level and a trend graph, e.g. an eight-hour trend graph, to the reader. CGM/FGM relies on the measurement of GL in the interstitial fluid in subcutaneous tissue, i.e. it does not measure blood GL, but in most scenarios, the levels have an accuracy > 80%. CGM sample a GL every $5^{th}$ minute, whereas an FGM samples GL every $15^{th}$ minute but also allows for the user to scan additional GL in between these 15-minute intervals. This results in 288 data points every day from a CGM and minimum of 96 data points from an FGM. The technology has improved the everyday life for many patients with T1D and insulin-treated type 2 diabetes thanks to their convenience of use. However, the vast amount of data generated from CGM/FGM is difficult to handle for the individual patient and even more so for the healthcare system since the amount of accumulated CGM/FGM-data is ever increasing. Given that most patients visit the clinic only once or twice every year this means that the patient will bring between 20,000-100,000 data points of just glucose data to each visit. The composite of this data reflects the individual risk of long-term complications since it describes the actual GL over time. In addition, hidden in this vast amount of data lies numerous readings of hypoglycemic events that could as mentioned, potentially be fatal. Despite the widespread use of CGM/FGM technology in routine care the utilization of the data is still low. In part this can be explained by the fact that CGM/FGM-data to a large extent needs to be interpreted manually and even consensus statements rely on interpretations primarily based on simple measurements such as time spent within target GL. Currently, the target levels for glucose control are normally solely based on HbA1c levels (HbAlc is also known as glycosylated, or glycated hemoglobin), a blood sample that roughly represents the average blood GL over the last 8-12 weeks. However, HbA1c levels do not resemble the fluctuations in blood GL, and in addition, there are a number of individual discrepancies in HbA1c levels. Considering the unmet need from the healthcare system and the costs related to the use of CGM/FGM, methods need to be developed that make better use of the collected data.

Examples of presently applied technology are disclosed in US-10321859 and in US-10373519. US-10321859 discloses systems and methods for capturing and analyzing hypoglycemic event data in order to provide feedback to a patient. US-10373519 discloses a system and method for determining and providing activity recommendations including to receive glucose level data and activity data. The objective is to provide recommendations to a user to encourage a greater number of times activities are undertaken.

Further examples of presently applied technology are disclosed in the following documents.

WO2015/025187A1 that discloses a method and device for improving prediction and detection of change in a physiological condition. This document is regarded as the closest prior art.

US2013/035871A1 that relates to system and methods for detecting glycose level data patterns.

US2016/354543A1 that relates to multivariable artificial pancreas method and system. US2015/347698A1 disclosing hazard based assessment patterns.

Anonymous: "Outlier", Wikipedia, 18 Dec. 2019, XP055698794, retrieved from the Internet: URL:https://en. wikipedia.org/w/index.php?title=Outlier&oldid=931339528. This citation describes the meaning of "outlier" in statistics.

D. M. NATHAN ET AL: "Translating the A1C Assay Into Estimated Average Glucose Values", DIABETES CARE, vol. 31, no. 8, 7 June 2008, pages 1473-1478, XP055099114, ISSN: 0149-5992, DOI: 10.2337/dc08-0545. This document discusses background technology.

Patent publication WO2018175935A1 is the closest prior art and discloses a system that analyzes relationships between entities, where one entity may correspond to an event entity (such as a meal event or an exercise event) and another corresponds to a glucose excursion event entity (such as a hypoglycemic event or a hyperglycemic event.

The object of the present invention is to achieve an improved diabetes analysis system and an improved method capable of performing automated analysis and interpretations of a large amount of collected data, e.g. CGM/FGM-data, and also capable of generating a root cause analysis and recommendations for treatment modifications for diabetes patients. In particular, the object is to achieve an improved system and method that will be a valuable tool for health care professionals (HCP) in order to improve diabetes care.

Summary

The above-mentioned objects are achieved by the present invention according to the independent claims.
Preferred embodiments are set forth in the dependent claims.
According to a first aspect the present invention relates to a diabetes analysis system for analysis and interpretation of data related to glucose level (GL) in blood, the system is to be applied to determine a treatment recommendation to a patient. The analysis system comprises:

- an input module (2) configured to receive GL related data (4) from measurements of interstitial fluid in subcutaneous tissue and prepare the data, e.g. by removing numerical outliers. The system further comprises:

  - a hypoglycemia identification module (6) configured to identify hypoglycemic events by performing a computer-implemented automatic search of said received GL related data, wherein all uninterrupted glucose levels less than a predetermined level, e.g. glucose levels < 3.5 mmol/L, in the same time series will be considered as one hypoglycemic event,
  - a hypoglycemia classification module (8) configured to analyze, for each identified hypoglycemic event, the glucose data during a predetermined first time period, e.g. three hours, preceding the hypoglycemic event, to determine the glucose level during the first time period, wherein the hypoglycemia classification module is configured to determine the type (10) of hypoglycemia event, based upon the glucose level during the first time period, by applying a computer-implemented pattern search procedure on a predetermined hypoglycemic classification scheme including different types of hypoglycemia, in order to identify the underlying cause of hypoglycemia.

According to a second aspect the present invention relates to a computer-implemented method for analysis and interpretation of data related to glucose level (GL) in blood, the **method** is to be applied to determine a treatment recommendation to a patient. The method comprises:

- receiving GL related data from measurements of interstitial fluid in subcutaneous tissue and preparing the data, e.g. by removing numerical outliers,
- identifying, in a hypoglycemia identification module, hypoglycemic events by performing a computer-implemented automatic search of said received GL related data, wherein all uninterrupted glucose levels less than a predetermined level, e.g. glucose levels < 3.5 mmol/L, in the same time series will be considered as one hypoglycemic event, and
- analyzing, in a hypoglycemia classification module, for each identified hypoglycemic event, the glucose data during a predetermined first time period, e.g. three (3) hours, preceding the hypoglycemic event, to determine the glucose level during the first time period, and determining the type of hypoglycemia event, based upon the glucose level during the first time period, by applying a computer-implemented pattern search procedure on a predetermined hypoglycemic classification scheme including different types of hypoglycemia, in order to identify the underlying cause of hypoglycemia.

The diabetes analysis system, and the method, according to the above aspects, are advantageous as they provide powerful tools for handling and analyzing a large amount of data related to the glucose level in blood obtained during days, weeks, or months.

Brief description of the drawings

Figure 1 is a block diagram schematically illustrating the present invention.
Figure 2 is a flow diagram illustrating the method according to the present invention.
Figure 3 is a block diagram schematically illustrating an embodiment of the present invention.

Figure 4 is histogram showing glucose profiles according to an embodiment of the present invention.

Figure 5 is a 3D histogram illustrating combined GL and dGL histograms according to an embodiment of the present invention.

Figure 6 is a hypoglycaemia score illustrating an exemplary output from a hypoglycaemia score module.

Figure 7 is a hyperglycaemia score illustrating an exemplary output from a hyperglycaemia score module.

Figure 8 is an overview flow diagram illustrating implementation of the system and method of the present invention.

Detailed description

The diabetes analysis system, and the computer-based method, will now be described in detail with references to the appended figures. Throughout the figures, the same, or similar, items have the same reference signs. Moreover, the items and the figures are not necessarily to scale, emphasis instead being placed upon illustrating the principles of the invention.

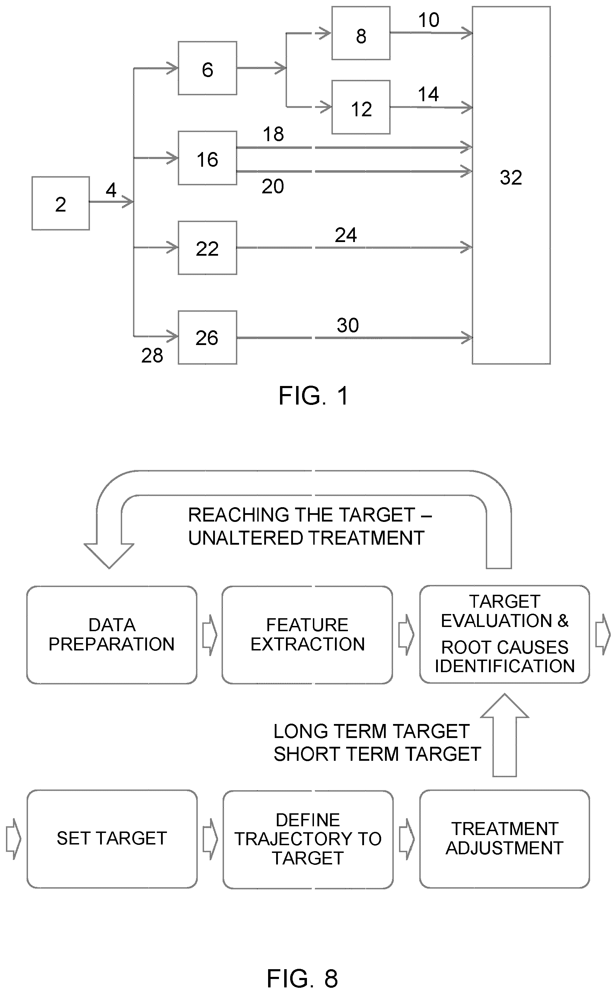

With references to the schematic block diagram shown in figure 1 a diabetes analysis system for analysis and interpretation of data related to glucose level (GL) in blood will be described in detail. The system is to be applied to determine a treatment recommendation for a patient. The analysis system comprises an input module 2 configured to receive GL related data from measurements of interstitial fluid in the subcutaneous tissue of a patient.

The diabetes analysis system further comprises a hypoglycemia identification module 6 configured to identify hypoglycemic events by performing a computer-implemented automatic search of the received GL related data, wherein all uninterrupted glucose levels less than a predetermined level, e.g. glucose levels < 3.5 mmol/L, in the same time series, will be considered as one hypoglycemic event. Glucose data of the identified hypoglycemic events is then applied to a hypoglycemia classification module 8 configured to analyze, for each identified hypoglycemic event, the glucose data during a predetermined first time period, e.g. three hours, preceding the hypoglycemic event, to determine the glucose level during the first time period. The hypoglycemia classification module is then configured to determine the type 10 of hypoglycemia event, based upon the glucose level during the first time period, by applying a computer-implemented pattern search procedure on a predetermined hypoglycemic classification scheme including different types of hypoglycemia, in order to identify the underlying cause of hypoglycemia.

The above-described identification and classification of hypoglycemic events are performed on GL related data obtained during days, weeks, or months, e.g. during the whole time period between follow-up meetings with the responsible physician.

According to an embodiment, the hypoglycemia identification module 6 is further configured to determine the duration of the hypoglycemic event, based upon time series of data of an identified hypoglycemic event, and also the severity of the event which is based upon the lowest recorded glucose value.

According to another embodiment, the diabetes analysis system also comprises a hypoglycemia recoil classification module 12 configured to analyze, for each identified hypoglycemic event, the glucose data during a predetermined second time period, e.g. two hours, following the hypoglycemic event, to determine the glucose level during the second time period. The hypoglycemia recoil classification module is configured determine the type 14 of hypoglycemia recoil, based upon the glucose level during the second time period, by applying a computer-implemented pattern search procedure on a predetermined hypoglycemic recoil classification scheme including different types of hypoglycemia recoil.

According to still another embodiment the diabetes analysis system comprises a cluster identification module 16 configured to receive GL values and arranged them as the numbers of GL samples in a time series that exist in specific GL intervals, denoted bins, to determine a GL histogram profile 18 of a patient during a predetermined time period, e.g. one week, and configured to determine a rate of change of glucose level (dGL) histogram profile 20 of a patient during said predetermined time period, e.g. one week.

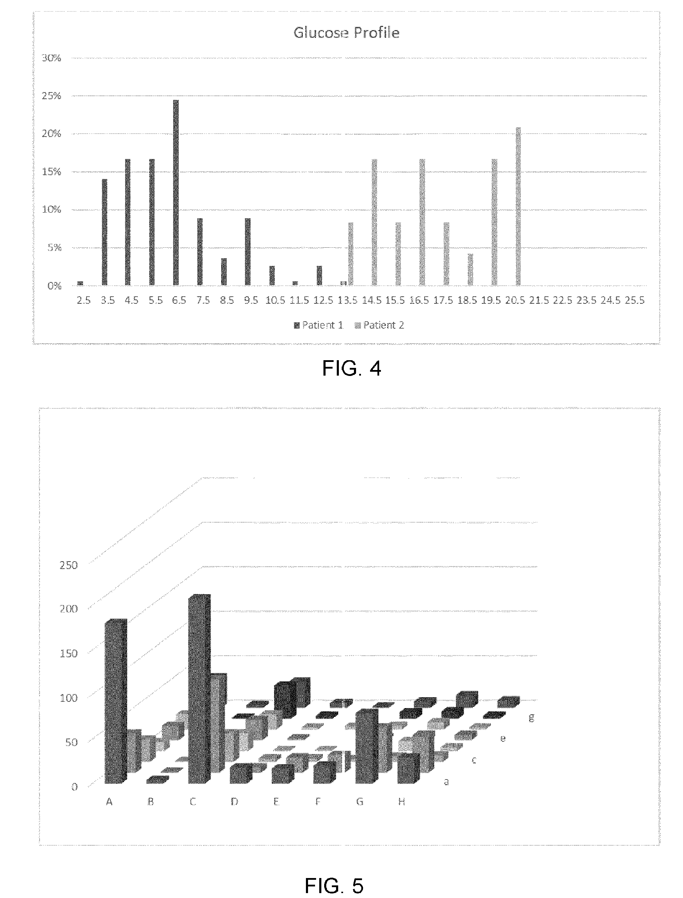

In particular, the GL histogram profile 18 comprises a horizontal axis showing bins of GL value intervals and a vertical axis e.g. showing duration in hours in each bin, or percentage of time interval for the histogram analysis in each bin, i.e. in each GL value interval, during the predetermined time period. The dGL histogram profile 20 comprises a horizontal axis showing bins of dGL value intervals and a vertical axis showing duration in hours in each bin, i.e. in each dGL value interval, during the predetermined time period. Examples of GL histograms for two patients are illustrated in figure 4.

In one embodiment, the cluster identification module 16 is configured to apply a computer-implemented procedure to compare said GL and dGL histogram profiles 18, 20 to sets of a predetermined number, e.g. 8, different GL type profiles (A-H) and dGL type profiles (a-h), respectively. This is performed in order to determine which GL type profile and dGL type profile that essentially corresponds to the determined GL histogram profile and dGL histogram profile, respectively, and to combine said thus determined GL and dGL type profiles to a classification (A-H, a-h) and insert the classification in a classification space, wherein each position in said classification has designated treatment schemes. An illustrating example of the classification space is shown in figure 5.

According to another embodiment the diabetes support system comprises a prandial event module 22

comprising a prandial event filter configured to detect meals for a patient without knowledge on bolus insulin and carbohydrate registrations. The prandial event filter is adapted determine a prandial event by identifying predefined curve shapes of the GL related data, and to classify the identified prandial events by applying a prandial classification based on pattern recognition, and wherein a basal insulin pressure is identified based upon said classified prandial event 24.

A prandial event is preferably detected by detecting a steep rising response of the GL as a consequence of the intake of carbohydrates. The higher glycemic index the carbohydrates have, the steeper the response will be, and the time for the start of the meal may be detected by applying three conditions:

Conditions that have to be fulfilled to detect a prandial event:

$$Forward4dGL = \sum_{0}^{3} dGL(i)$$

$$Backward4dGL = \sum_{-3}^{0} dGL(i)$$

$$Forward4dGL > 0 \text{ AND } Forward4dGL > 3\ Backward4dGL$$

$$\text{Then } t0 = t_{prandial}$$

This means that the detection of prandial events is based on comparing the rate of change for GL forward and backward from the possible time of the prandial event.

The prandial event detection monitors the rate of change in GL in a window which is e.g. 3 samples forward and 3 samples backward. If a significant and persistent change occurs, this time is considered to be a candidate for a prandial event. Data is then stored in a vector of candidate events. The second step is a classification of the candidate events to be a real prandial event or not. This is done by using e.g. a random forest methodology where a set of events are classified by clinicians. Ideally, real event registrations with insulin injection time, insulin type and carbohydrates ingested are used to train the system. After training, the classification system will filter out real prandial events from the gross list of candidates. The prandial events are used to identify pre- and post-prandial event GL, the prandial delta, as well as analyzing the basal pressure, i.e. the effect of the basal insulin on the metabolic balance when no bolus insulin nor any meals are included.

The pre-prandial GL is defined as the GL(0), i.e. time of the event. The post-prandial GL is defined as the GL(+3hours). Prandial delta is calculated as GL(+3hours)-GL(0).

Based on the prandial events, the basal insulin pressure can be identified. I.e. the rate of change of GL without the impact of bolus insulin given at the meals. The basal insulin pressure is used as an input to the treatment recommendation.

According to still another embodiment, the diabetes analysis system also comprises an estimated hemoglobin (HbA1c) module 26. The input module 2 is configured to receive HbAlc related data 28 and to apply the data to the estimate HbA1c module 26. This module is configured to determine estimated HbA1c data 30 based upon the HbA1c related data, and then to apply the estimated HbA1c data to a diabetes analysis module 32 that is configured to combine the determined estimated HbA1c data to the determined type 10 of hypoglycemia event, and to at least one of type 14 of hypoglycemia recoil, and **determined** GL and dGL type profiles.

The GL related data 4 preferably comprises data from continuous glucose measurements (CGM) and/or flash glucose measurements (FGM). Also, any other type of measurement device configured to determine glucose levels, e.g. transdermal sensors, may naturally be applied.

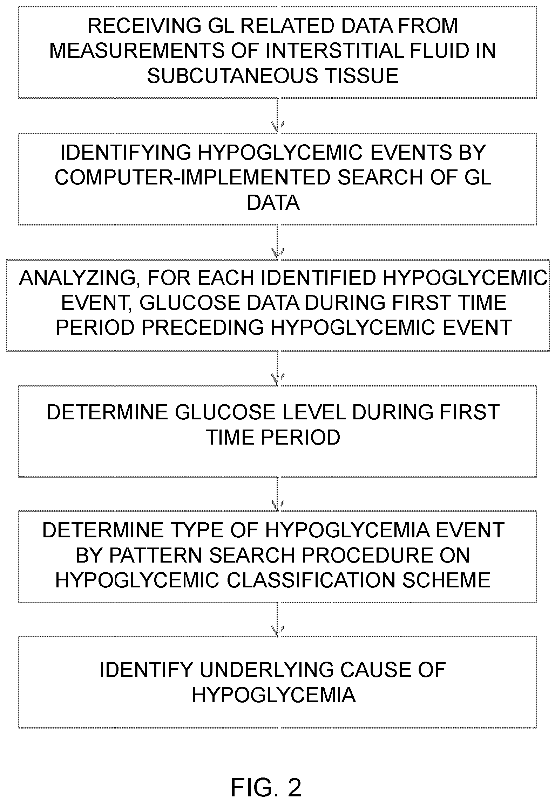

The present invention also relates to a computer-implemented method for analysis and interpretation of data related to glucose level (GL) in blood. The method will be described with references to the flow diagram shown in figure 2.

The method is to be applied to determine a treatment recommendation to a patient and is to be implemented by the diabetes analysis system described above. In the following, some parts of the system will be described, but it is also referred to the description above, and also below, of the various parts of the system.

The method comprises:

- receiving GL related data from measurements of interstitial fluid in subcutaneous tissue.
- identifying, in a hypoglycemia identification module, hypoglycemic events by performing a computer-implemented automatic search of said received GL related data, wherein all uninterrupted glucose levels less than a predetermined level, e.g. glucose levels < 3.5 mmol/L, in a same time series will be considered as one hypoglycemic event, and

- analyzing, in a hypoglycemia classification module, for each identified hypoglycemic event, the glucose data during a predetermined first time period, e.g. three (3) hours, preceding the hypoglycemic event, to determine the glucose level during the first time period, and determining the type of hypoglycemia event, based upon the glucose level during the first time period, by applying a computer-implemented pattern search procedure on a predetermined hypoglycemic classification scheme including different types of hypoglycemia, in order to identify the underlying cause of hypoglycemia.

Preferably, the method also comprises determining the duration of the hypoglycemic event, based upon time series of data of an identified hypoglycemic event, and to determine the severity of the event which is based upon the lowest recorded glucose value.

In a further embodiment, the method comprises:

- analyzing, in a hypoglycemia recoil classification module, for each identified hypoglycemic event, the glucose data during a predetermined second time period, e.g. two (2) hours, following the hypoglycemic event, to determine the glucose level during the second time period, and determining the type of hypoglycemia recoil, based upon the glucose level during the second time period, by applying a computer-implemented pattern search procedure on a predetermined hypoglycemic recoil classification scheme including different types of hypoglycemia recoil.

According to another embodiment the computer-implemented method also comprises:

- receiving, in a cluster identification module, GL values and arranging them as the numbers of GL samples in a time series that exist in specific GL intervals, denoted bins, determining, based upon said GL values, a GL histogram profile of a patient during a predetermined time period, e.g. one week, and determining a rate of change of glucose level (dGL) histogram profile of a patient during said predetermined time period, e.g. one week. Further information of the GL and dGL histogram profiles are discussed above in relation to the description of the system.

Preferably, the computer-implemented method also comprises applying a computer-implemented procedure to compare the GL and dGL histogram profiles to sets of a predetermined number, e.g. 8, different GL type profiles (A-H) and dGL type profiles (ah), respectively, in order to determine which GL type profile and dGL type profile that essentially corresponds to the determined GL histogram profile and dGL histogram profile, respectively. The method further comprises to combine said thus determined GL and dGL type profiles to a classification (A-H, a-h) and insert the classification in a classification space, wherein each position in said classification has designated treatment schemes.

In another embodiment, the computer-implemented method comprises:

- receiving HbA1c related data by the input module,
- determining estimated HbA1c data, in an estimate hemoglobin (HbA1c) module, and
- combining, in a diabetes analysis module 32, said determined estimated HbA1c data to at least one of the determined type 10 of hypoglycemia event, type 14 of hypoglycemia recoil, and determined GL and dGL type profiles.

The GL related data advantageously comprises data from continuous glucose measurements (CGM) and/or flash glucose measurements (FGM). Also other types of measurement devices may be applied, e.g. transdermal sensors.

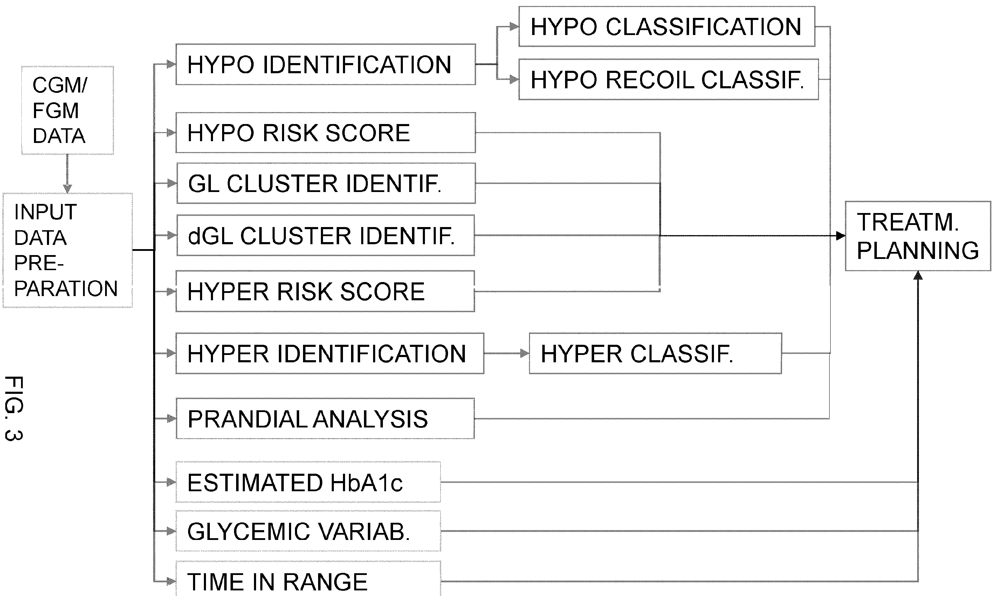

In figure 3 is shown a schematic block diagram of a diabetes analysis system according to one variation of the present invention. In the block diagram some blocks are included that have been described above. In addition, further blocks are shown that optionally may be part of the system.

The flow starts with the patient uploading data from FGM/CGM and other devices such as activity trackers etc. In this, the consistency of data is checked to remove possible outlier samples, i.e. samples that are considered to be statistically unlikely. The filter for removing outliers is cautious not to remove low values since these are analyzed by the hypo identification and classification module. As an example, if sampling time is 15 min, this will result in 4x24 = 96 samples per day, which is 672 samples per week.

The blocks in figure 3 will now be described. Some of the blocks/modules described in the following have also been discussed above.

**Estimated HbA1c module**

Hemoglobin A1c, often abbreviated HbA1c, is a form of hemoglobin (a blood protein that carries oxygen) that is bound to glucose. A blood test for HbA1c level is routinely performed in people with type 1 and type 2 diabetes mellitus. Blood HbA1c levels are reflective of how well diabetes is controlled. To estimate the HbA1c a mean value of the blood glucose is used as an input to a function that maps the mean value onto an HbA1c value where any length of the time window may be selected for the calculation of the mean value, e.g. day, week, or month.

Thus, the system comprises an HbA1c determining module configured to determine an estimated HbA1c level and to generate an HbA1c level signal.

**Glycemic variability module**

Glycemic variability (GV), which refers to swings in blood glucose levels, has a broader meaning because it alludes to blood glucose oscillations that occur throughout the day, including hypoglycemic periods and postprandial increases, as well as blood glucose fluctuations that occur at the same time on different days.

The broad definition of GV considers the intraday glycemic excursions, including episodes of hyperglycemia and hypoglycaemia, and may be determined according to the following expression.

$$GV = \frac{\sum_1^n \sqrt{(dGL_i^2 + \Delta t^2)}}{\sum_1^n \Delta t} - 1$$

**Time in range module**

Time-in-Range (TIR) is the percentage of time that a person spends with their blood glucose levels in a target range. The range will vary depending on the person, but general guidelines suggest starting with a range of 4 to 10 mmol/l.

Time-above-Range (TAR) is the percentage of time that a person spends with their blood glucose levels above a target range.

Time-below-Range (TBR) is the percentage of time that a person spends with their blood glucose levels below a target range.

**Hypoglycemia identification and classification module**

Hypoglycemia (normally defined as GL < 3.5 mmol/l) occurs due to an imbalance of insulin doses in relation to carbohydrate intake and can be augmented by a number of external and physiological factors such as physical activity for instance. A smaller number of patients record all injected insulin doses and carbohydrates ingested, in that case, the hypoglycemic events can easily be explained and diagnosed. However, most patients do not record insulin doses at all or only a minority of the doses and therefore it is difficult to analyze the cause of hypoglycemic events in the clinical setting. In addition, the number of recorded hypoglycemic events even for a single patient from the last 3-6 months can easily exceed 350 events making it impossible to analyze manually at a clinical visit. By an automated search of the complete data set, all GL < 3.5 mmol/l are identified. All uninterrupted levels < 3.5 mmol/L in the same time series will be considered as one hypoglycemic event. Based on the hypoglycemic time series of data two different analysis are performed:

1. The duration of the hypoglycemic event.
2. The severity of the event which is based upon the lowest recorded glucose value.

In addition, for each hypoglycemic episode the glucose data is analyzed during e.g. the three (3) hours preceding the first identified hypoglycemic GL. Based on the GL from this time series, the underlying cause of hypoglycemia can be identified even without knowledge of insulin doses and carbohydrate intake. This is achieved by employing a training set of hypoglycemic events identified in both patients which have registered insulin doses and those without registered insulin doses.

In setting up the system, a number of hypoglycemic events (without presentation of insulin doses) is then selected by a stratification algorithm and partly overlapping events are presented to clinical experts for classification. The independent classification of each expert is analyzed firstly for conformity in between evaluators and next the same experts are presented with the remaining hypoglycemic events, but in this case with the data on insulin doses and carbohydrate intake. The classifications on the complete data set is then checked for conformity in-between evaluators but also compared with the first classification made by a peer without knowledge on insulin doses and carbohydrate intake.

After this two-step process of classification, the data will be used to train the machine-learning algorithm, the algorithm will be programmed to incorporate data on insulin doses and carbohydrate intake if available. A cluster-sample of algorithm derived classification based on GL alone will again be analyzed for conformity by clinical experts with the addition of insulin and carbohydrate data. The interpretations of the algorithm will automatically generate a probability rating and a cut-off value, based on the comparison with clinical experts, will be defined.

If heart rate data is available this will be used in the classification in order to identify exercise-induced hypoglycemic events. A scenario specific for CGM/FGM is the possibility of false hypoglycemic recordings due to mechanical pressure of the sensor which reduce the interstitial fluid flow creating a local hypoglycemic environment which does not reflect the systemic GL, most commonly observed during sleep. Data on concomitant drugs known to increase the risk of hypoglycemia (for instance opioids, benzodiazepines and indirectly sodium-glucose transport protein2 inhibitors) will be collected and if the patient is using any of such medications this will be highlighted as a contributor if there are no other apparent causes. In a small subset of patients with T1D (<5%) GL can be extremely volatile, also known as brittle-diabetes. In that situation the underlying cause for hypoglycemia will most likely not be identified and in that case the event will be assigned the term "unclassified" and highlighted for the HCP to interpret manually in dialog with the patient.

Example of hypoglycemic classifications are e.g.:

- High basal insulin pressure
- Overcompensation of hyperglycemia
- Overestimated bolus insulin at meal
- Exercise resulting in hypoglycemia (combined CGM/FGM data with heart rate and physical activity data)
- False hypoglycemia due to mechanical pressure on the sensor
- Contribution of concomitant drugs
- Unclassified (less than 5% of all hypoglycemic episodes)

The type of hypoglycemic event is identified by a random forest learner/predictor or any other similar method. After the training, the predictor will process a large set of data and assign conformity and probability to each event. By combining self-scanned GL for all patients who use an FGM it can also be identified whether the patient was aware of the event and provide more detailed data on how he/she handles it. For instance, does the frequency of self-scanned GL increases in the coming hours after a hypoglycemic event when compared to the average number of scanning.

Thus, herein is described how to achieve a classification of hypoglycemia, including providing a classification by combining input from experts with automated recognition procedures. The thus obtained hypoglycemia classification is then applied for classifying data for a patient.

**Hypo recoil identification/classification module**

The hypo recoil identification module works in the same way as the identification and classification of hypoglycemic events. When the hypoglycemic event has been resolved, i.e. GL have returned to > 3.5 mmol/l the following window of e.g. two (2) hours is analyzed to identify if there is recoil in GL. The recoil may not exist or can be an effect of over ingestion by the patient in the ambition to get the GL back to normal or due to a counter-regulatory hormonal response (glucagon, cortisol, adrenaline, and noradrenaline release).

Data from the two-hour window after the hypoglycemic event is used to train the random forest learner/predictor or any other similar method. A set of training events is selected by a stratification algorithm. This set is then classified by clinical expertise in the same fashion as for the hypo identification system described above. For the hypo recoil classification module, data from individuals that register carbohydrate intake will be used for validation of the interpretation. Also for this parameter heart rate data, if available, will be used in the interpretation since a more pronounced increase in heart rate will be associated with a hormonally based recoil due to the release of adrenaline and noradrenaline. Example of such classifications are e.g.:

- No recoil

- Mild recoil

- Recoil due to over ingestion

- Hormonal based recoil (absence of manual readings during hypoglycemic period increase the likelihood of hormonal recoil)

- Recoil leading to hyperglycemia

After the training, the predictor will process a large set of data and assign conformity to each event. In addition, if a recoil of GL is identified (regardless of the cause) the combined contribution on daily average GL and estimated HbA1c levels is analyzed by combining the hypoglycemic event (lowering average- and estimated HbA1c) and the recoil phase (may last for hours and increase the average- and estimated HbA1c level).

From this analysis, a hypoglycemic driven increase in mean blood GL can be identified.

**Hypo identification, classification and recoil classification.**

By identifying all hypoglycemic events, trends and patterns over time and days can be analyzed. Hence, certain "windows in time" with a high presence of hypoglycemic events can be identified. These could, in turn, be explained by certain behaviors or routines in everyday life which will have to be addressed in dialog with the patient. Based on the systematic classification of hypoglycemic events it can be identified the root cause in relation to the current insulin treatment which can be used as a basis for treatment recommendations. For example, if a patient displays frequent hypoglycemic episodes at night due to a too high basal pressure of insulin the recommendation would be to reduce the basal insulin dose. If instead there are frequent hypoglycemic episodes due to corrective insulin boluses administered when the GL was above target range, the first recommendation would be to avoid corrective doses and instead work with the underlying cause of the high GL, which could be due to a low basal insulin pressure. If a patient with frequent hypoglycemic events use a concomitant drug known to increase the risk of hypoglycemia it would be suggested to the HCP to find an alternative treatment without this side-effect. In this analysis it is also interpreted self-scanned GL from patients who use an FGM. These data points give a unique insight into whether the patient is aware of the event and if so how he/she handles it. Similarly, by identifying recoil GL following a hypoglycemic event it can be provided feedback on the amount of carbohydrates consumed to counteract a hypoglycemic episode. In addition, it can be estimated the impact of the hypoglycemic episode and the following recoil and provide feedback on how much the metabolic control could be improved by avoiding hypoglycemic events.

**Hypo risk score module**

The hypo risk score is calculated by the hypo risk score module as a combination of the actual GL value as well as the rate of change of the glucose level dGL. Each sample of GL will enable a score and these scores are summarized by parts of a day, by day or over an individually specified period of time.
The cut-off values for both GL and dGL need to be tuned but the principles are:

- A low GL that is falling yields a high-risk score
- A normal GL that is falling yields a moderate risk

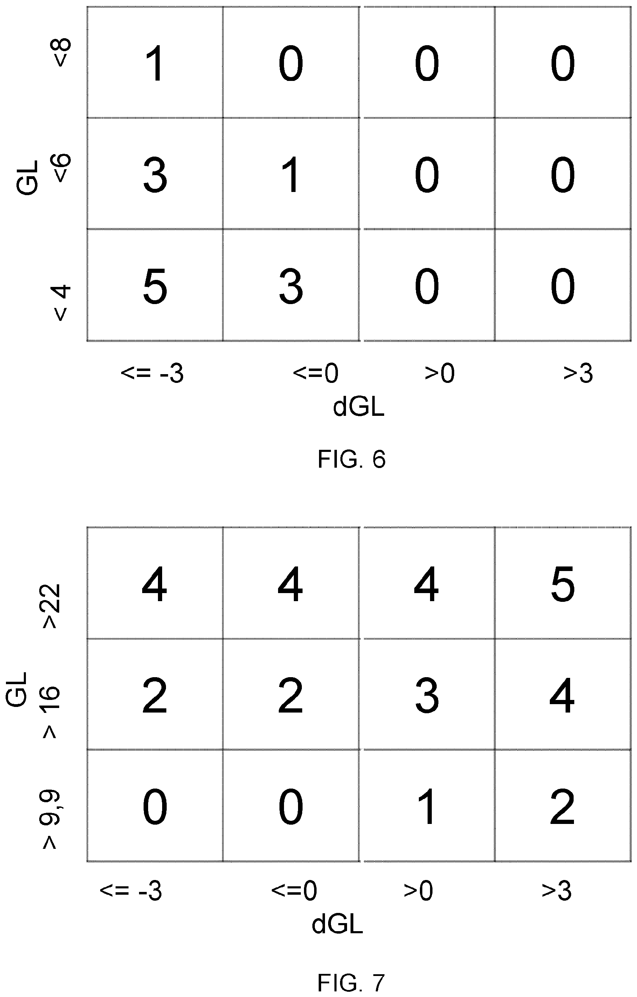

By calculating a hypoglycemic risk score it can, based on an individual number, be classified the glycemic profile and the overall risk of hypoglycemic events. A high-risk score is often related to high variability in GL, which often is caused by inadequate basal insulin pressure and frequent corrective insulin boluses. On the other hand, in a patient with poor metabolic control (i.e. high estimated HbA1c and average GL) and a low hypo risk score the treatment recommendations can be more aggressive in terms of percentage increase of both basal- and bolus insulin doses. In figure 6 is shown one example of indicating the hypo risk score. The score is based upon assessing both the absolute GL value as well as the rate of change dGL. The risk score is highest in case of a low GL and a dGL that indicates a quick decrease of GL. The cut off values given in the illustrated score are just examples and will be parameters that will be tuned with input from clinical practice.

**Hyper risk score module**

The hyper risk score is calculated by the hyper risk score module as a combination of the actual GL value as well as the rate of change dGL. Each sample of GL will enable a score and these scores are summarized by parts of a day, by day or over an individually specified period of time.
The cut-off values for both GL and dGL need to be tuned but the principles are:

- A very high GL that continues rising yields a very high-risk score
- A very high GL that is fluctuating yields a high-risk score
- A high GL that is rising yields a moderate risk
- A GL in the upper normal range which is increasing yields a mild risk score

The hyper risk score is based on all episodes with a rapid increase in GL and is a valuable tool much like the hypo risk score in order to summarize the overall risk in one value. High levels of hyper risk score are often related to inadequate insulin boluses at meals which will result in a rapid increase in GL. Another common cause is ingestion of carbohydrate snacks without administering insulin. In figure 7 is shown one example of indicating the hypo risk score. The score is based on assessing both the absolute GL value as well as the rate of change dGL. The risk score is highest in the case of a high GL and a dGL that indicates a quick increase of GL. The illustrated cut off values are just examples and will be parameters that will be tuned with input from clinical practice.

**Prandial event module**

A prandial event filter in the prandial event module is configured to detect meals without knowledge on bolus insulin and carbohydrate registrations. If such registrations are available this step is not needed but, as stated above, most patients do not consistently register insulin doses and carbohydrate intake. This module has been described in detail above.

A pre- and post-prandial filter is provided that is configured to identify meals without knowledge of insulin administration and carbohydrate intake. In most CGM/FGM, it is possible to register insulin doses and carbohydrates consumed, but most patients do not register all meals and/or insulin doses. For large scale analysis, it is, therefore, crucial to be able to identify these events without this data input. Based on this analysis the algorithm can systematically evaluate the accuracy of insulin bolus doses at each meal based on the GL prior to the meal compared to that two (2) hours after a meal, which ideally should be equal. Based on this analysis the algorithm can directly advise on how to adjust the bolus insulin doses. By excluding all prandial events from the daily curve the algorithm can evaluate the effect of the basal insulin dose which is the main regulator of GL under fasting conditions. By applying this filter the algorithm can hence give recommendations on both bolus- and basal insulin doses.

**Cluster analysis**

The cluster analysis is based on histograms of both the GL and the dGL. A histogram is a graph that displays the number of samples from a time series that exists in a specific interval, i.e. a bin. In that way, a large number of samples can be summarized in one graph. In this case, the number of samples in each bin has been replaced by time in bin or percentage of time in the specific analysis period. In the case described herein, each sample is Ts (0.1-15 min) and the time in a specific bin is measured in hours.

It is currently identified 8 clusters of GL profiles, but the number of clusters is not constant and may increase as more patient data are entered. The bin size is set to 1 mmol/L and named of the midpoint. E.g. interval 4 to 5 is named 4.5. The GL clusters are named A, B,..., H with capital letters and in the order of rising mean GL. Both the GL distribution and the dGL distribution may be clustered using a k-nearest neighbour algorithm, or any other applicable algorithm.

The dGL is analyzed in two ways:

1) With the same histogram analysis as the GL.
2) With measures of the statistical distribution.

The latter is the method that is currently used.

From a clinical perspective it is mainly two characteristics that are useful: How many samples represent a GL that is quickly falling or rising, i.e. the tails of the distribution, and how the center is skewed towards rising or falling. Therefore, in one exemplary implementation, for the dGL distribution the Skewness and Kurtosis are calculated. Skewness is a measure of symmetry, or more precisely, the lack of symmetry. A distribution, or data set, is symmetric if the left and right part of the center point are equal. Kurtosis is a measure of whether the data are heavy-tailed or light-tailed relative to a normal distribution. The "patient-time intervals" (day-weeks-months) are clustered in 8 dGL profiles with increasing kurtosis and varying skewness. The dGL clusters are named a, b, c...h where the h cluster has the highest dGL values, i.e. highest kurtosis. Also other algorithms may be applied, but it is also possible to use the bin-density directly.

The combination of GL and dGL cluster forms a position in the classification space. A patient in e.g. [C,a] has a tight distribution of GL and small variations of dGL. On the other hand, a patient in [H,h] has a very problematic metabolic control with GL over the whole range and very fast swings from low values to high values. In figure 5 one illustrative example of a classification space showing combined GL and dGL clusters.

The clinical conclusion is that a patient cannot improve the GL control without first reducing the dGL thus forming the trajectory towards improved glycemic control. This presents a preferred direction of improvement for patients with poor glycemic control, starting with reducing the variations before the mean values are reduced.

Each combination in the space will also be associated with a set of root causes of why metabolic control is not optimal.

The classification space of GL and dGL may be extended to the third dimension with the same kind of histogram for physical activity.

### GL and dGL Cluster Identification

The combination of GL and dGL cluster forms a position in the classification space. A patient in e.g. [C,a] has a tight distribution of GL and small variations of dGL. On the other hand, a patient in [H,h] has a very problematic metabolic control with GL over the whole range and very fast swings from low values to high values. Each position in the classification space is associated with one or several underlying root causes and are presented in general terms to the patient and HCP, and based on all other parameters analyzed, the most important underlying root cause for that individual will be identified. In addition, the classification space provides a trajectory and plan or improvement.

Providing a two-dimensional cluster classification space defined by clusters of GL and dGL, respectively, where each cluster is a histogram including a number of events that fulfills the requirements of that cluster, comprising a predetermined number of clusters each having different GL/dGL profiles, e.g. 8 clusters of different GL/dGL profiles.

### Treatment planning - diabetes analysis module

Based on the automated analysis the most likely root causes for suboptimal glucose control are identified. Underlying root causes can include too high or low basal insulin pressure, hypoglycemic episodes due to repeated correction insulin doses, too high or low insulin doses at meals, and much more. The underlying root causes will be systematically evaluated based on some or all parameters included in the block diagram shown in figure 3.

By analyzing a large number of patients and weeks and classifying these weeks into clusters like [C,a] and [B,d] we can identify subgroups of patients with different profiles or signatures. The estimated HbA1c is calculated for each patient and the average HbA1c is calculated for each cluster combination, i.e. 8x8= 64 sets of patient weeks. Not all combinations are possible and the movements in the classification space over time follow specific patterns, much like the predefined moves in chess where the moves for different checkers are predefined. Based on these large scale analyses a database of patient recorded GL and matching GL and dGL clusters have been built. By using the database the most suitable trajectory towards an improved metabolic control may be identified based on the movement in the classification space that has been observed for other patients. This is like finding a path in the forest where the straight-line from point X to point Y is usually not the most appropriate path. By using the collected experience from our database on how patients can move in the classification space it can be forecasted the most efficient trajectory which will be presented to the HCP and in turn to the patient.

The treatment recommendations will be a summary of all individual recommendations generated by each parameter of analysis. These will be weighted together in order to give a conform recommendation.

The analysis of most importance for the treatment recommendation is the combination of GL and dGL cluster identification with the estimated HbA1c level. In current clinical practice, HbA1c levels are used as a single measure of treatment goals but it does not take into account the level of glucose excursions and hypoglycemic events. Hence, a patient can currently be classified as optimally treated despite experiencing volatile glucose excursions and frequent hypoglycemic episodes. By combining the estimated HbA1c levels with the cluster identification defined herein the bar will be raised for the treatment goals and be able to quickly identify patients with a high degree of glucose variability even if the estimated HbA1c is within the target range.

Finally, with references to figure 8, an overall workflow will be discussed involving the system and method described above.

The layout of the overall workflow loops each time a patient meets with his/her health care professionals (HCP) with new analysis, understanding the current situation and planning the next steps towards the long term goal.

The flow starts with the patient uploading data from FGM/CGM and other devices such as activity trackers etc. In this, the consistency of data is checked to remove possible outlier samples. In the following step the essential features are calculated to facilitate subsequent machine learning steps. In machine learning, pattern recognition, and in image processing, feature extraction starts from an initial set of measured data and builds derived values (features) intended to be informative and non-redundant, facilitating the subsequent learning and generalization steps that will enable improved interpretations of the data.

In the following step, the features are analyzed together with the treatment target to find root causes behind any deviations from the target. If there is no deviation, the immediate recommendation will be an unaltered treatment recommendation, and the following process steps are omitted.

If there is a deviation between the treatment target and the current situation, the inferred root causes are used to define a short-term target that is realistic to reach within a defined time of follow-up which is a step on the path towards the long-term target, i.e. optimal glucose control with a high degree of flexibility. Based on the short-term target the trajectory for the improvements to address is defined. Finally, the treatment adjustment proposals based on the short-target are derived and presented to the HCP along with the proposed trajectory towards the fulfilment of the long-term target. These adjustments proposed to the HCP will have to be approved based on clinical judgment before presented to the patient. The HCP can also modify the treatment plan if he/she finds this suitable, these modifications will be stored for future augmentation of the algorithm after approval of a scientific board.

Thus, in order to fully utilize the potential of CGM/FGM in diabetes care, it has been developed a diabetes analysis system that can generate automated interpretations and, in addition, generate recommendations for treatment modifications. The system will be a much needed and valuable tool for HCP in order to improve diabetes care.

The present invention is defined by the appended claims.

**Claims**

1. Diabetes analysis system for analysis and interpretation of data related to glucose level (GL) in blood, the system is to be applied to determine a treatment recommendation to a patient, the analysis system comprises:

   - an input module (2) configured to receive GL related data (4) from measurements of interstitial fluid in subcutaneous tissue and prepare the data, e.g. by removing numerical outliers, **characterized in that** said system further comprises:

      - a hypoglycemia identification module (6) configured to identify hypoglycemic events by performing a computer-implemented automatic search of said received GL related data, wherein all uninterrupted glucose levels less than a predetermined level in the same time series will be considered as one hypoglycemic event, and that said predetermined level is e.g. 3.5 mmol/L,
      - a hypoglycemia classification module (8) configured to analyze, for each identified hypoglycemic event, the glucose data during a predetermined first time period, e.g. three hours, preceding the hypoglycemic event, to determine the glucose level during the first time period, wherein the hypoglycemia classification module is configured to determine the type (10) of hypoglycemia event, based upon the glucose level during the first time period, by applying a computer-implemented pattern search procedure on a predetermined hypoglycemic classification scheme including different types of hypoglycemia, in order to identify the underlying cause of hypoglycemia-, wherein said different types of hypoglycemia includes at least one of:

         - High basal insulin pressure,
         - Overcompensation of hyperglycemia,
         - Overestimated bolus insulin at meal,
         - Exercise resulting in hypoglycemia (combined CGM/FGM data with heart rate and physical activity data),
         - False hypoglycemia due to mechanical pressure on the sensor,
         - Contribution of concomitant drugs,
         - Unclassified (less than 5% of all hypoglycemic episodes).

2. The diabetes analysis system according to claim 1, wherein the hypoglycemia identification module (6) is further configured to determine the duration of the hypoglycemic event, based upon time series of data of an identified hypoglycemic event, and the severity of the event which is based upon the lowest recorded glucose value,

3. The diabetes analysis system according to claim 1 or 2, comprising a hypoglycemia recoil classification module (12) configured to analyze, for each identified hypoglycemic event, the glucose data during a predetermined second time period, e.g. two hours, following the hypoglycemic event, to determine the glucose level during the second time period, wherein the hypoglycemia recoil classification module is configured determine the type (14) of hypoglycemia recoil, based upon the glucose level during the second time period, by applying a computer-implemented pattern search procedure on a predetermined hypoglycemic recoil classification scheme including different types of hypoglycemia recoil.

4. The diabetes analysis system according to any of claims 1-3, comprising a cluster identification module (16) configured to receive GL values and arranged them as the numbers of GL samples in a time series that exist in specific GL intervals, denoted bins, to determine a GL histogram profile (18) of a patient during a predetermined time period, e.g. one week, and configured to determine a rate of change of glucose level (dGL) histogram profile (20) of a patient during said predetermined time period, e.g. one week,

5. The diabetes analysis system according to claim 4, wherein said cluster identification module (16) is configured to apply a computer-implemented procedure to compare said GL and dGL histogram profiles (18, 20) to sets of a predetermined number, e.g. 8, different GL type profiles (A-H) and dGL type profiles (a-h), respectively, to determine which GL type profile and dGL type profile that essentially corresponds to the determined GL histogram profile and dGL histogram profile, respectively, and to combine said thus determined GL and dGL type profiles to a classification (A-H, a-h) and insert the classification in a classification space, wherein each position in said classification has designated treatment schemes.

6. The diabetes support system according to any of claims 1-5, comprising a prandial event module (22) comprising a prandial event filter configured to detect meals for patient without knowledge on bolus insulin and carbohydrate registrations, wherein the prandial event filter is adapted determine a prandial event by identifying predefined curve shapes of said GL related data, and to classify said identified prandial events by applying a prandial classification based on pattern recognition, and wherein a basal insulin pressure is identified based upon said classified prandial event (24).

7. The diabetes analysis system according to any of claims 1-6, comprising an estimate hemoglobin (HbA1c) module (26), wherein said input module (2) is configured to receive HbA1c related data (28) and said estimate HbA1c module is configured to determine estimated HbA1c data (30) based upon said HbA1c related data, and to apply said estimated HbA1c data to a diabetes analysis module (32) that is configured to combine said determined estimated HbA1c data to at least one of the determined type (10) of hypoglycemia event, type (14) of hypoglycemia recoil, and determined GL and dGL type profiles.

8. The diabetes analysis system according to any of claims 1-7, wherein said GL related data (4) comprises data from continuous glucose measurements (CGM) and/or flash glucose measurements (FGM).

9. A computer-implemented method for analysis and interpretation of data related to glucose level (GL) in blood, the method is to be applied to determine a treatment recommendation to a patient, and comprises:

- receiving GL related data from measurements of interstitial fluid in subcutaneous tissue and preparing the data, e.g. by removing numerical outliers, **characterized in that** said method further comprises:

- identifying, in a hypoglycemia identification module, hypoglycemic events by performing a computer-implemented automatic search of said received GL related data, wherein all uninterrupted glucose levels less than a predetermined level in the same time series will be considered as one hypoglycemic event, and that said predetermined level is e.g. 3.5 mmol/L,
- analyzing, in a hypoglycemia classification module, for each identified hypoglycemic event, the glucose data during a predetermined first time period, e.g. three (3) hours, preceding the hypoglycemic event, to determine the glucose level during the first time period, and determining the type of hypoglycemia event, based upon the glucose level during the first time period, by applying a computer-implemented pattern search procedure on a predetermined hypoglycemic classification scheme including different types of hypoglycemia, in order to identify the underlying cause of hypoglycemia-, wherein said different types of hypoglycemia includes at least one of:

- High basal insulin pressure,
- Overcompensation of hyperglycemia,
- Overestimated bolus insulin at meal,
- Exercise resulting in hypoglycemia (combined CGM/FGM data with heart rate and physical activity data),
- False hypoglycemia due to mechanical pressure on the sensor,
- Contribution of concomitant drugs,
- Unclassified (less than 5% of all hypoglycemic episodes).

10. The computer-implemented method according to claim 9, comprising determining the duration of the hypoglycemic event, based upon time series of data of an identified hypoglycemic event, and the severity of the event which is based upon the lowest recorded glucose value.

11. The computer-implemented method according to claim 9 or 10, comprising:

- analyzing, in a hypoglycemia recoil classification module, for each identified hypoglycemic event, the glucose data during a predetermined second time period, e.g. two (2) hours, following the hypoglycemic event, to determine the glucose level during the second time period, and determining the type of hypoglycemia recoil, based upon the glucose level during the second time period, by applying a computer-implemented pattern search procedure on a predetermined hypoglycemic recoil classification scheme including different types of hypoglycemia recoil.

12. The computer-implemented method according to any of claims 9-11, comprising:

- receiving, in a cluster identification module, GL values and arranging them as the numbers of GL samples in a time series that exist in specific GL intervals, denoted bins, determining, based upon said GL values, a GL histogram profile of a patient during a predetermined time period, e.g. one week, and determining a rate of change of glucose level (dGL) histogram profile of a patient during said predetermined time period, e.g. one week.

13. The computer-implemented method according to claim 12, comprising applying a computer-implemented procedure to compare said GL and dGL histogram profiles to sets of a predetermined number, e.g. 8, different GL type profiles (A-H) and dGL type profiles (a-h), respectively, to determine which GL type profile and dGL type profile that essentially corresponds to the determined GL histogram profile and dGL histogram profile, respectively, and to combine said thus determined GL and dGL type profiles to a classification (A-H, a-h) and insert the classification in a classification space, wherein each position in said classification has designated treatment schemes.

14. The computer-implemented method according to any of claims 9-13, comprising:

- receiving HbA1c related data by said input module,
- determining estimated HbA1c data, in an estimate hemoglobin (HbA1c) module, and
- combining, in a diabetes analysis module (22), said determined estimated HbA1c data to at least one of the determined type (10) of hypoglycemia event, type (14) of hypoglycemia recoil, and determined GL and dGL type profiles.

15. The computer-implemented method according to any of claims 9-14, wherein said GL related data comprises data from continuous glucose measurements (CGM) and/or flash glucose measurements (FGM).

**Patentansprüche**

1. Diabetesanalysesystem zur Analyse und Interpretation von Daten, die sich auf den Glukosespiegel (GL) im Blut beziehen, wobei das System dazu dient, eine Behandlungsempfehlung für einen Patienten zu bestimmen, wobei das Analysesystem Folgendes umfasst:

- ein Eingabemodul (2), das dazu ausgestaltet ist, GL-bezogene Daten (4) aus Messungen von interstitieller Flüssigkeit in subkutanem Gewebe zu empfangen und die Daten aufzubereiten, z. B. durch Entfernen numerischer Ausreißer, **dadurch gekennzeichnet, dass** das System ferner Folgendes umfasst:

- ein Hypoglykämieidentifizierungsmodul (6), das dazu ausgestaltet ist, hypoglykämische Ereignisse durch Durchführen einer computerimplementierten automatischen Suche der empfangenen GL-bezogenen Daten zu identifizieren, wobei alle ununterbrochenen Glukosespiegel unterhalb eines vorbestimmten Spiegels in derselben Zeitreihe als ein hypoglykämisches Ereignis betrachtet werden und wobei der vorbestimmte Spiegel z. B. 3,5 mmol/L beträgt,
- ein Hypoglykämieklassifizierungsmodul (8), das dazu ausgestaltet ist, für jedes identifizierte hypoglykämische Ereignis die Glukosedaten während eines vorbestimmten ersten Zeitraums, z. B. drei Stunden, vor dem hypoglykämischen Ereignis zu analysieren, um den Glukosespiegel während des ersten Zeitraums zu bestimmen, wobei das Hypoglykämieklassifizierungsmodul dazu ausgestaltet ist, den Typ (10) des Hypoglykämieereignisses auf der Grundlage des Glukosespiegels während des ersten Zeitraums durch Anwenden eines computerimplementierten Mustersuchvorgangs auf ein vorbestimmtes Hypoglykämieklassifizierungsschema, das verschiedene Hypoglykämietypen umfasst, zu bestimmen, um die zugrunde liegende Ursache der Hypoglykämie zu identifizieren, wobei die verschiedenen Hypoglykämietypen mindestens einen der folgenden umfassen:

- hoher Basalinsulindruck,
- Überkompensation von Hyperglykämie,
- überschätztes Bolusinsulin bei der Mahlzeit,

- Belastung, die zu einer Hypoglykämie führt (kombinierte CGM/FGM-Daten mit Herzfrequenzdaten und Daten körperlicher Aktivität),
- falsche Hypoglykämie aufgrund von mechanischem Druck auf den Sensor,
- Beitrag von Begleitmedikamenten,
- nicht klassifiziert (weniger als 5 % aller hypoglykämischer Episoden).

2. Diabetesanalysesystem nach Anspruch 1, wobei das Hypoglykämieidentifizierungsmodul (6) ferner dazu ausgestaltet ist, die Dauer des hypoglykämischen Ereignisses auf der Grundlage von Zeitreihen von Daten eines identifizierten hypoglykämischen Ereignisses und den Schweregrad des Ereignisses, der auf dem niedrigsten aufgezeichneten Glukosewert basiert, zu bestimmen.

3. Diabetesanalysesystem nach Anspruch 1 oder 2, umfassend ein Hypoglykämierückstoßklassifizierungsmodul (12), das dazu ausgestaltet ist, für jedes identifizierte hypoglykämische Ereignis die Glukosedaten während eines vorbestimmten zweiten Zeitraums, z. B. zwei Stunden, nach einem hypoglykämischen Ereignis zu analysieren, um den Glukosespiegel während des zweiten Zeitraums zu bestimmen, wobei das Hypoglykämierückstoßklassifizierungsmodul dazu ausgestaltet ist, den Typ (14) des Hypoglykämierückstoßes auf der Grundlage des Glukosespiegels während des zweiten Zeitraums durch Anwenden eines computerimplementierten Mustersuchvorgangs auf ein vorbestimmtes Hypoglykämierückstoßklassifizierungsschema, das verschiedene Hypoglykämierückstoßtypen umfasst, zu bestimmen.

4. Diabetesanalysesystem nach einem der Ansprüche 1 bis 3, umfassend ein Clusteridentifizierungsmodul (16), das dazu ausgestaltet ist, GL-Werte zu empfangen und sie als die Zahlen von GL-Proben in einer Zeitreihe anzuordnen, die in spezifischen - als Bins bezeichneten - GL-Intervallen vorhanden sind, um ein GL-Histogrammprofil (18) eines Patienten während eines vorbestimmten Zeitraums, z. B. einer Woche, zu bestimmen, und das dazu ausgestaltet ist, ein Histogrammprofil (20) der Änderungsrate des Glukosespiegels (dGL) eines Patienten während des vorbestimmten Zeitraums, z. B. einer Woche, zu bestimmen.

5. Diabetesanalysesystem nach Anspruch 4, wobei das Clusteridentifizierungsmodul (16) dazu ausgestaltet ist, einen computerimplementierten Vorgang anzuwenden, um das GL- und das dGL-Histogrammprofil (18, 20) mit Sätzen einer vorbestimmten Zahl, z. B. 8, verschiedener GL-Typ-Profile (A-H) bzw. dGL-Typ-Profile (a-h), zu vergleichen, um zu bestimmen, welches GL-Typ-Profil und dGL-Typ-Profil im Wesentlichen dem bestimmten GL-Histogrammprofil bzw. dGL-Histogrammprofil entspricht, und um die so bestimmten GL- und dGL-Typ-Profile zu einer Klassifikation (A-H, a-h) zu kombinieren und die Klassifikation in einen Klassifikationsraum einzufügen, wobei jede Position in der Klassifikation zugewiesene Behandlungsschemata aufweist.

6. Diabetesunterstützungssystem nach einem der Ansprüche 1 bis 5, umfassend ein Prandiales-Ereignis-Modul (22), das einen Prandiales-Ereignis-Filter umfasst, der dazu ausgestaltet ist, Mahlzeiten für einen Patienten ohne Kenntnis von Bolusinsulin- und Kohlenhydratregistrierungen zu erkennen, wobei der Prandiales-Ereignis-Filter dazu ausgelegt ist, ein prandiales Ereignis durch Identifizieren vordefinierter Kurvenformen der GL-bezogenen Daten zu bestimmen und die identifizierten prandialen Ereignisse durch Anwenden einer Prandialklassifizierung auf der Grundlage von Mustererkennung zu klassifizieren, und wobei ein Basalinsulindruck auf der Grundlage des klassifizierten prandialen Ereignisses (24) identifiziert wird.

7. Diabetesanalysesystem nach einem der Ansprüche 1 bis 6, umfassend ein Schätzungs-Hämoglobin(HbA1c)-Modul (26), wobei das Eingabemodul (2) dazu ausgestaltet ist, HbAlcbezogene Daten (28) zu empfangen, und das Schätzungs-HbA1c-Modul dazu ausgestaltet ist, geschätzte HbA1c-Daten (30) auf der Grundlage der HbAlc-bezogenen Daten zu bestimmen und die geschätzten HbAlc-Daten auf ein Diabetesanalysemodul (32) anzuwenden, das dazu ausgestaltet ist, die bestimmten geschätzten HbAlc-Daten mit mindestens einem des bestimmten Hypoglykämieereignisstyps (10), des Hypoglykämierückstoßtyps (14) und der ermittelten GL- und dGL-Typ-Profile zu kombinieren.

8. Diabetesanalysesystem nach einem der Ansprüche 1 bis 7, wobei die GL-bezogenen Daten (4) Daten aus kontinuierlichen Glukosemessungen (CGM) und/oder Flash-Glukosemessungen (FGM) umfassen.

9. Computerimplementiertes Verfahren zur Analyse und Interpretation von Daten, die sich auf den Glukosespiegel (GL) im Blut beziehen, wobei das Verfahren dazu dient, eine Behandlungsempfehlung für einen Patienten zu bestimmen, und Folgendes umfasst:

- Empfangen von GL-bezogenen Daten aus Messungen von interstitieller Flüssigkeit in subkutanem Gewebe und Aufbereiten der Daten, z. B. durch Entfernen numerischer Ausreißer, **dadurch gekennzeichnet, dass** das Verfahren ferner Folgendes umfasst:

- Identifizieren hypoglykämischer Ereignisse in einem Hypoglykämieidentifizierungsmodul durch Durchführen einer computerimplementierten automatischen Suche der empfangenen GL-bezogenen Daten, wobei alle ununterbrochenen Glukosespiegel unterhalb eines vorbestimmten Spiegels in derselben Zeitreihe als ein hypoglykämisches Ereignis betrachtet werden und wobei der vorbestimmte Spiegel 3,5 mmol/L beträgt,

- Analysieren der Glukosedaten während eines vorbestimmten ersten Zeitraums, z. B. drei (3) Stunden, vor dem hypoglykämischen Ereignis in einem Hypoglykämieklassifizierungsmodul für jedes identifizierte hypoglykämische Ereignis, um den Glukosespiegel während des ersten Zeitraums zu bestimmen, und Bestimmen des Hypoglykämieereignistyps auf der Grundlage des Glukosespiegels während des ersten Zeitraums durch Anwenden eines computerimplementierten Mustersuchvorgangs auf ein vorbestimmtes Hypoglykämieklassifizierungsschema, das verschiedene Typen von Hypoglykämie umfasst, um die zugrunde liegende Ursache der Hypoglykämie zu identifizieren, wobei die verschiedenen Hypoglykämietypen mindestens einen der folgenden umfassen:

- hoher Basalinsulindruck,
- Überkompensation von Hyperglykämie,
- überschätztes Bolusinsulin bei der Mahlzeit,
- Belastung, die zu einer Hypoglykämie führt (kombinierte CGM/FGM-Daten mit Herzfrequenzdaten und Daten körperlicher Aktivität),
- falsche Hypoglykämie aufgrund von mechanischem Druck auf den Sensor,
- Beitrag von Begleitmedikamenten,
- nicht klassifiziert (weniger als 5 % aller hypoglykämischer Episoden).

10. Computerimplementiertes Verfahren nach Anspruch 9, umfassend Bestimmen der Dauer des hypoglykämischen Ereignisses auf der Grundlage von Zeitreihen von Daten eines identifizierten hypoglykämischen Ereignisses und des Schweregrads des Ereignisses, der auf dem niedrigsten aufgezeichneten Glukosewert basiert.

11. Computerimplementiertes Verfahren nach Anspruch 9 oder 10, umfassend:

- Analysieren der Glukosedaten während eines vorbestimmten zweiten Zeitraums, z. B. zwei (2) Stunden, nach dem hypoglykämischen Ereignis in einem Hypoglykämierückstoßklassifizierungsmodul für jedes identifizierte hypoglykämische Ereignis, um den Glukosespiegel während des zweiten Zeitraums zu bestimmen, und Bestimmen des Hypoglykämierückstoßtyps auf der Grundlage des Glukosespiegels während des zweiten Zeitraums durch Anwenden eines computerimplementierten Mustersuchvorgangs auf ein vorbestimmtes Hypoglykämierückstoßklassifizierungsschema, das verschiedene Hypoglykämierückstoßtypen umfasst.

12. Computerimplementiertes Verfahren nach einem der Ansprüche 9 bis 11, umfassend:

- Empfangen von GL-Werten in einem Clusteridentifizierungsmodul und Anordnen derselben als Zahlen von GL-Proben in einer Zeitreihe, die in spezifischen - als Bins bezeichneten - GL-Intervallen vorhanden sind, Bestimmen eines GL-Histogrammprofils eines Patienten während eines vorbestimmten Zeitraums, z. B. einer Woche, auf der Grundlage der GL-Werte, und Bestimmen eines Histogrammprofils der Änderungsrate des Glukosespiegels (dGL) eines Patienten während des vorbestimmten Zeitraums, z. B. einer Woche.

13. Computerimplementiertes Verfahren nach Anspruch 12, umfassend das Anwenden eines computerimplementierten Vorgangs, um das GL- und das dGL-Histogrammprofil mit Sätzen einer vorbestimmten Zahl, z. B. 8, verschiedener GL-Typ-Profile (A-H) bzw. dGL-Typ-Profile (a-h) zu vergleichen, um zu bestimmen, welches GL-Typ-Profil und dGL-Typ-Profil im Wesentlichen dem bestimmten GL-Histogrammprofil bzw. dGL-Histogrammprofil entspricht, und um die so bestimmten GL- und dGL-Typ-Profile mit einer Klassifikation (A-H, a-h) zu kombinieren und die Klassifikation in einen Klassifikationsraum einzufügen, wobei jede Position in der Klassifikation zugewiesene Behandlungsschemata aufweist.

14. Computerimplementiertes Verfahren nach einem der Ansprüche 9 bis 13, umfassend:

- Empfangen von HbA1c-bezogenen Daten durch das Eingabemodul,
- Bestimmen geschätzter HbAlc-Daten in einem Schätzungs-Hämoglobin(HbA1c)-Modul und
- Kombinieren der bestimmten geschätzten HbAlc-Daten mit mindestens einem des bestimmten Hypoglykämieereignisstyps (10), des Hypoglykämierückstoßtyps (14) und der bestimmten GL- und dGL-Typ-Profile.

15. Computerimplementiertes Verfahren nach einem der Ansprüche 9 bis 14, wobei die GL-bezogenen Daten Daten aus kontinuierlichen Glukosemessungen (CGM) und/oder Flash-Glukosemessungen (FGM) umfassen.

**Revendications**

1. Système d'analyse du diabète pour l'analyse et l'interprétation de données relatives au taux de glucose (GL) dans le sang, le système étant à appliquer pour déterminer une recommandation de traitement à un patient, le système d'analyse comprenant :

   - un module d'entrée (2) configuré pour recevoir des données relatives à GL (4) à partir de mesures de fluide interstitiel dans un tissu sous-cutané et préparer les données, par exemple en éliminant des valeurs aberrantes numériques, **caractérisé en ce que** ledit système comprend en outre :

   - un module d'identification d'hypoglycémie (6) configuré pour identifier des événements hypoglycémiques en effectuant une recherche automatique mise en œuvre par ordinateur desdites données liées à GL reçues, dans lequel tous les niveaux de glucose ininterrompus inférieurs à un niveau prédéterminé dans la même série temporelle seront considérés comme un événement hypoglycémique, et que ledit niveau prédéterminé est par exemple de 3,5 mmol/L,
   - un module de classification de l'hypoglycémie (8) configuré pour analyser, pour chaque événement hypoglycémique identifié, les données de glucose pendant une première période de temps prédéterminée, par ex., trois heures, précédant l'événement hypoglycémique, pour déterminer le taux de glucose pendant la première période de temps, dans lequel le module de classification d'hypoglycémie est configuré pour déterminer le type (10) d'événement d'hypoglycémie, sur la base du niveau de glucose pendant la première période de temps, en appliquant une procédure de recherche de motif implémentée par ordinateur sur un schéma de classification hypoglycémique prédéterminé comprenant différents types d'hypoglycémie, afin d'identifier la cause sous-jacente de l'hypoglycémie-, dans lequel lesdits différents types d'hypoglycémie comprennent au moins un parmi :

      - Pression d'insuline basale élevée,
      - Surcompensation de l'hyperglycémie,
      - Bolus d'insuline surestimé au repas,
      - Exercice entraînant une hypoglycémie (données CGM/FGM combinées avec des données de fréquence cardiaque et d'activité physique),
      - Fausse hypoglycémie due à une pression mécanique sur le capteur,
      - Contribution des médicaments concomitants,
      - Non classé (moins de 5 % de tous les épisodes hypoglycémiques).

2. Système d'analyse de diabète selon la revendication 1, dans lequel le module d'identification d'hypoglycémie (6) est en outre configuré pour déterminer la durée de l'événement hypoglycémique, sur la base d'une série temporelle de données d'un événement hypoglycémique identifié, et la gravité de l'événement qui est basé sur la valeur de glucose enregistrée la plus basse,

3. Système d'analyse du diabète selon la revendication 1 ou 2, comprenant un module de classification de recul d'hypoglycémie (12) configuré pour analyser, pour chaque événement hypoglycémique identifié, les données de glucose pendant une deuxième période de temps prédéterminée, par ex., deux heures, suite à l'événement hypoglycémique, pour déterminer le taux de glucose pendant la deuxième période de temps, dans lequel le module de classification de recul d'hypoglycémie est configuré pour déterminer le type (14) de recul d'hypoglycémie, sur la base du niveau de glucose pendant la deuxième période de temps, en appliquant une procédure de recherche de motif implémentée par ordinateur sur un schéma de classification de recul hypoglycémique prédéterminé comprenant différents types de recul hypoglycémique.

4. Système d'analyse du diabète selon l'une quelconque des revendications 1 à 3, comprenant un module d'identification de grappe (16) configuré pour recevoir des valeurs GL et les agencer en tant que nombres d'échantillons GL dans une série temporelle qui existent dans des intervalles GL spécifiques, les bacs désignés, pour déterminer un profil d'histogramme GL (18) d'un patient pendant une période de temps prédéterminée, par ex., une semaine, et configuré pour déterminer un profil d'histogramme de taux de changement de niveau de glucose (dGL) (20) d'un patient pendant ladite période de temps prédéterminée, par ex., une semaine,

5. Système d'analyse du diabète selon la revendication 4, dans lequel ledit module d'identification de grappe (16) est configuré pour appliquer une procédure implémentée par ordinateur pour comparer lesdits profils d'histogramme GL et dGL (18, 20) à des ensembles d'un nombre prédéterminé, par ex., 8, différents profils de type GL (A-H) et profils de type dGL (a-h), respectivement, pour déterminer quel profil de type GL et quel profil de type dGL correspondent essentiellement au profil d'histogramme GL et au profil d'histogramme dGL déterminés, respectivement, et à combiner lesdits profils de type GL et dGL ainsi déterminés à une classification (A-H, a-h) et insérer la classification dans un espace de classification, dans lequel chaque position dans ladite classification a des schémas de traitement désignés.

6. Système de support pour le diabète selon l'une quelconque des revendications 1 à 5, comprenant un module d'événement prandial (22) comprenant un filtre d'événement prandial configuré pour détecter des repas pour un patient sans connaissance des enregistrements d'insuline et de glucides en bolus, dans lequel le filtre d'événement prandial est adapté pour déterminer un événement prandial en identifiant des formes de courbes prédéfinies desdites données liées à GL, et pour classer lesdits événements prandiaux identifiés en appliquant une classification prandiale basée sur la reconnaissance de formes, et dans lequel une pression d'insuline basale est identifiée sur la base dudit événement prandial classifié (24).

7. Système d'analyse du diabète selon l'une quelconque des revendications 1 à 6, comprenant un module (26) d'estimation de l'hémoglobine (HbAlc), dans lequel ledit module d'entrée (2) est configuré pour recevoir des données liées à l'HbAlc (28) et ledit module d'estimation de l'HbAlc est configuré pour déterminer des données d'HbAlc estimées (30) sur la base desdites données liées à l'HbAlc, et d'appliquer lesdites données d'HbAlc estimées à un module d'analyse de diabète (32) qui est configuré pour combiner lesdites données d'HbAlc estimées déterminées à au moins un du type déterminé (10) d'événement d'hypoglycémie, type (14) de recul de l'hypoglycémie, et les profils déterminés de type GL et dGL.

8. Système d'analyse du diabète selon l'une quelconque des revendications 1 à 7, dans lequel lesdites données relatives à GL (4) comprennent des données provenant de mesures de glucose continues (CGM) et/ou de mesures de glucose flash (FGM).

9. L'invention concerne un procédé informatique d'analyse et d'interprétation de données relatives au taux de glucose (GL) dans le sang, le procédé étant destiné à être appliqué pour déterminer une recommandation de traitement à un patient, et comprenant :

   - la réception de données liées à la GL à partir de mesures de fluide interstitiel dans un tissu sous-cutané et la préparation des données, par exemple en éliminant des valeurs aberrantes numériques, **caractérisé en ce que** ledit procédé comprend en outre :

      - l'identification, dans un module d'identification d'hypoglycémie en effectuant une recherche automatique mise en œuvre par ordinateur desdites données liées à GL reçues, dans lequel tous les niveaux de glucose ininterrompus inférieurs à un niveau prédéterminé dans la même série temporelle seront considérés comme un événement hypoglycémique, et que ledit niveau prédéterminé est par exemple de 3,5 mmol/L,
      - l'analyse, dans un module de classification de l'hypoglycémie, pour chaque événement hypoglycémique identifié, les données de glucose pendant une première période de temps prédéterminée, par ex., trois (3) heures, précédant l'événement hypoglycémique, pour déterminer le taux de glucose pendant la première période de temps, et détermination du type d'événement d'hypoglycémie, sur la base du niveau de glucose pendant la première période de temps, en appliquant une procédure de recherche de motif mise en œuvre par ordinateur sur un schéma de classification hypoglycémique prédéterminé comprenant différents types d'hypoglycémie, afin d'identifier la cause sous-jacente de l'hypoglycémie-, dans lequel lesdits différents types d'hypoglycémie comprennent l'au moins un parmi
      - Pression d'insuline basale élevée,
      - Surcompensation de l'hyperglycémie,
      - Bolus d'insuline surestimé au repas,

- Exercice entraînant une hypoglycémie (données CGM/FGM combinées avec des données de fréquence cardiaque et d'activité physique),
- Fausse hypoglycémie due à une pression mécanique sur le capteur,
- Contribution des médicaments concomitants,
- Non classé (moins de 5 % de tous les épisodes hypoglycémiques).

10. Procédé mis en œuvre par ordinateur selon la revendication 9, comprenant la détermination de la durée de l'événement hypoglycémique, sur la base d'une série temporelle de données d'un événement hypoglycémique identifié, et la gravité de l'événement qui est basée sur la valeur de glucose enregistrée la plus basse.

11. Procédé mis en œuvre par ordinateur selon la revendication 9 ou 10, comprenant :

- l'analyse, dans un module de classification du recul de l'hypoglycémie, pour chaque événement hypoglycémique identifié, les données de glucose pendant une deuxième période de temps prédéterminée, par ex., deux (2) heures, suite à l'événement hypoglycémique, pour déterminer le taux de glucose pendant la deuxième période de temps, et détermination du type de recul de l'hypoglycémie, sur la base du niveau de glucose pendant la deuxième période de temps, en appliquant une procédure de recherche de motif mise en œuvre par ordinateur sur un schéma de classification de recul hypoglycémique prédéterminé comprenant différents types de recul hypoglycémique.

12. Procédé mis en œuvre par ordinateur selon la revendication 9 à 11, comprenant :

- la réception, dans un module d'identification de grappe, des valeurs de GL et les organiser en tant que nombres d'échantillons de GL dans une série temporelle qui existent dans des intervalles de GL spécifiques, notés bacs, déterminer, sur la base desdites valeurs de GL, un profil d'histogramme de GL d'un patient pendant une période de temps prédéterminée, par exemple une semaine, et déterminer un taux de changement de niveau de glucose (dGL) profil d'histogramme d'un patient pendant ladite période de temps prédéterminée.

13. Procédé mis en œuvre par ordinateur selon la revendication 12, comprenant l'application d'une procédure mise en œuvre par ordinateur pour comparer lesdits profils d'histogramme GL et dGL à des ensembles d'un nombre prédéterminé, par ex., 8, différents profils de type GL (A-H) et profils de type dGL (a-h), respectivement, pour déterminer quel profil de type GL et quel profil de type dGL correspondent essentiellement au profil d'histogramme GL et au profil d'histogramme dGL déterminés, respectivement, et à combiner lesdits profils de type GL et dGL ainsi déterminés à une classification (A-H, a-h) et insérer la classification dans un espace de classification, dans lequel chaque position dans ladite classification a des schémas de traitement désignés.

14. Procédé mis en œuvre par ordinateur selon la revendication 9 à 13, comprenant :

- la réception des données liées à la fonction HbAlc par ledit module d'entrée,
- la détermination des données de l'HbAlc estimées, dans un module d'hémoglobine estimée (HbAlc), et
- la combinaison, dans un module d'analyse du diabète (22), desdites données de l'HbAlc estimées déterminées à l'au moins un parmi le type déterminé (10) d'événement d'hypoglycémie, le type (14) de recul d'hypoglycémie, et des profils déterminés de type GL et dGL.

15. Procédé mis en œuvre par ordinateur selon l'une quelconque des revendications 9 à 14, dans lequel lesdites données relatives à GL comprennent des données provenant de mesures de glucose continues (CGM) et/ou de mesures de glucose flash (FGM).

FIG. 1

FIG. 8

RECEIVING GL RELATED DATA FROM MEASUREMENTS OF INTERSTITIAL FLUID IN SUBCUTANEOUS TISSUE

IDENTIFYING HYPOGLYCEMIC EVENTS BY COMPUTER-IMPLEMENTED SEARCH OF GL DATA

ANALYZING, FOR EACH IDENTIFIED HYPOGLYCEMIC EVENT, GLUCOSE DATA DURING FIRST TIME PERIOD PRECEDING HYPOGLYCEMIC EVENT

DETERMINE GLUCOSE LEVEL DURING FIRST TIME PERIOD

DETERMINE TYPE OF HYPOGLYCEMIA EVENT BY PATTERN SEARCH PROCEDURE ON HYPOGLYCEMIC CLASSIFICATION SCHEME

IDENTIFY UNDERLYING CAUSE OF HYPOGLYCEMIA

FIG. 2

FIG. 3

FIG. 4

FIG. 5

|  | <= -3 | <=0 | >0 | >3 |
|---|---|---|---|---|
| GL <8 | 1 | 0 | 0 | 0 |
| GL <6 | 3 | 1 | 0 | 0 |
| < 4 | 5 | 3 | 0 | 0 |

dGL

FIG. 6

|  | <= -3 | <=0 | >0 | >3 |
|---|---|---|---|---|
| GL >22 | 4 | 4 | 4 | 5 |
| GL > 16 | 2 | 2 | 3 | 4 |
| > 9,9 | 0 | 0 | 1 | 2 |

dGL

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10321859 B
- US 10373519 B
- WO 2015025187 A1
- US 2013035871 A1
- US 2016354543 A1
- US 2015347698 A1
- WO 2018175935 A1

**Non-patent literature cited in the description**

- Outlier. *Wikipedia*, 18 December 2019, https://en.wikipedia.org/w/index.php?title=Outlier&oldid=931339528
- **D. M. NATHAN et al.** Translating the A1C Assay Into Estimated Average Glucose Values. *DIABETES CARE*, 07 June 2008, vol. 31 (8), ISSN 0149-5992, 1473-1478